# EUROPÄISCHE PATENTANMELDUNG

(11) **EP 3 178 556 A1**
(43) Veröffentlichungstag der Anmeldung: **14.06.2017**
(21) Anmeldenummer: 15199277.3
(22) Anmeldetag: 10.12.2015
(51) Int. Cl.: B01L 3/00, G01N 33/49, G01N 21/03

(54) **KÜVETTE UND MESSVERFAHREN**

(71) Anmelder: Behnk, Holger, 22417 Hamburg (DE)
(72) Erfinder: Behnk, Holger, 22417 Hamburg (DE)
(74) Vertreter: Glawe, Delfs, Moll

(57) **Zusammenfassung**

Die Erfindung betrifft eine Küvette zum Mischen einer Flüssigkeit. Die Küvette umfasst einen Innenraum (18) zur Aufnahme der Flüssigkeit und eine in dem Innenraum (18) ausgebildete Laufschiene (20) für eine Kugel (22). Erfindungsgemäß wird die Laufschiene (20) von zwei Vorsprüngen (12, 13) gebildet, die von gegenüberliegenden Seitenwänden (10, 11) der Küvette (14) in den Innenraum (18) vorspringen. In der erfindungsgemäßen Küvette (14) lässt sich mit einer geringen Flüssigkeitsmenge ein hoher Füllstand erreichen, was für optische Messungen von Vorteil ist. Die Erfindung betrifft außerdem ein Messverfahren unter Verwendung der Küvette.

## Beschreibung

Die Erfindung betrifft eine zum Rühren einer Flüssigkeit geeignete Küvette. Die Küvette hat einen Innenraum zur Aufnahme der Flüssigkeit. In dem Innenraum ist eine Laufschiene für eine Kugel ausgebildet. Die Erfindung betrifft außerdem ein zugehöriges Verfahren.

Eine Flüssigkeit in einer Küvette kann gerührt werden, indem eine Kugel innerhalb der Flüssigkeit bewegt wird. Durch die Laufschiene im Innenraum der Küvette kann ein definierter Weg vorgegeben werden, entlang dem sich die Kugel beim Rühren bewegt.

Für bestimmte Anwendungen ist es wünschenswert, mit einer geringen Flüssigkeitsmenge einen hohen Füllstand in der Küvette zu erreichen. Dies gilt beispielsweise, wenn eine Analyse vorgenommen werden soll, bei der Licht durch die Flüssigkeit hindurch geleitet wird. Küvetten mit Laufschiene haben meist einen vergleichsweise großen Querschnitt. Es wird also eine größere Flüssigkeitsmenge benötigt, um eine bestimmte Füllhöhe zu erreichen.

Der Erfindung liegt die Aufgabe zugrunde, eine zum Rühren einer Flüssigkeit geeignete Küvette vorzustellen, bei der mit einer geringen Flüssigkeitsmenge eine große Füllhöhe erreicht wird. Ausgehend vom genannten Stand der Technik wird die Aufgabe gelöst mit den Merkmalen des Anspruchs 1. Vorteilhafte Ausführungsformen sind in den Unteransprüchen angegeben. Erfindungsgemäß wird die Laufschiene der Küvette von zwei Vorsprüngen gebildet, die von gegenüberliegenden Wänden der Küvette in den Innenraum vorspringen.

Indem die Laufschiene durch Vorsprünge gebildet wird, die von gegenüberliegenden Wänden der Küvette in den Innenraum vorspringen, wird es möglich, die Küvette schmal zu halten. Die Kugel kann also auf der Laufschiene entlang einem definierten Weg geführt werden, obwohl der Abstand zwischen den gegenüberliegenden Wänden kaum größer ist als der Durchmesser der Kugel.

Wenn die Kugel auf dem Boden der Küvette rollt, wird die Flüssigkeit beträchtlichen Druckkräften ausgesetzt. Bei empfindlichen Flüssigkeiten ist dies unerwünscht. Die Laufschiene ist deswegen vorzugsweise so angeordnet, dass die auf der Laufschiene rollende Kugel den Boden der Küvette nicht berührt. Für eine möglichst vollständige und gleichmäßige Durchmischung der Flüssigkeit ist es von Vorteil, wenn die Kugel nur einen geringfügigen Abstand zum Boden des Innenraums hat.

In einer bevorzugten Ausführungsform ist die Küvette deswegen so gestaltet, dass ein imaginärer erster Kreis, der senkrecht zur Laufschiene ausgerichtet ist und der am Boden des Innenraums sowie an zwei gegenüberliegenden Seitenwänden des Innenraums anliegt die beiden Vorsprünge schneidet. Weiter bevorzugt schneidet ein kleinerer, dazu konzentrischer imaginärer zweiter Kreis die Vorsprünge nicht. Der Durchmesser des zweiten Kreises ist mindestens 50 %, vorzugsweise mindestens 60 %, weiter vorzugsweise mindestens 80 % des Durchmessers des ersten Kreises.

Wo die Kugel auf der Laufschiene aufliegt, kann ein beträchtlicher Druck auf die Flüssigkeit wirken. Um die Beeinträchtigung der Flüssigkeit gering zu halten, ist die Laufschiene deswegen vorzugsweise so gestaltet, dass der Kontaktbereich zwischen der Laufschiene und der Kugel klein ist. Insbesondere kann der Vorsprung so gestaltet sein, dass er im Querschnitt betrachtet eine konvexe Form im Kontaktbereich hat. Der Vorsprung kann sich als geschlossene Form vom Kontaktbereich bis zum Boden der Küvette erstrecken. Es werden dadurch Hinterschneidungen vermieden, unter denen die Flüssigkeit eindringen kann.

Die beiden Vorsprünge können parallel zueinander ausgerichtet sein, sodass sich eine Laufschiene mit konstanter Breite ergibt. Die Laufschiene kann einen Abschnitt aufweisen, in dem sie sich geradlinig erstreckt. Die Laufschiene kann einen Abschnitt aufweisen, in dem sie sich parallel zum Boden der Küvette erstreckt. Die Laufschiene kann an einem Ende oder an beiden Enden nach oben umgelenkt sein. Durch eine solche Umlenkung nach oben kann verhindert werden, dass die Kugel an der Stirnwand der Küvette anstößt und damit lokal ein hoher Druck auf die Flüssigkeit ausgeübt wird. Der Übergang zwischen einem im Wesentlichen horizontalen Abschnitt der Laufschiene und dem nach oben umgelenkten Abschnitt ist vorzugsweise abgerundet, sodass die Kugel sanft abgebremst wird.

Die Seitenwände der Küvette können im Wesentlichen parallel zueinander sein. Die Seitenwände sind diejenigen Wände, an denen die Vorsprünge der Laufschiene ausgebildet sind. Im Wesentlichen parallel bedeutet, dass die Seitenwände bezogen auf die horizontale Dimension parallel zueinander ausgerichtet sind, während in der vertikalen Dimension eine leichte Aufweitung nach oben zulässig ist. Möglich ist, dass die Seitenwände auch in der vertikalen Dimension parallel zueinander ausgerichtet sind. Versuche haben gezeigt, dass es für den Mischvorgang förderlich ist, wenn die Seitenflächen nach oben hin leicht auseinander laufen. Beispielsweise können die Seitenflächen in der vertikalen Dimension einen Winkel zwischen 0,2° und 5°, vorzugsweise zwischen 0,5° und 2° einschließen. Alle Richtungsangaben beziehen sich auf den Zustand, in dem die Küvette über ihre Bodenfläche aufrecht auf einer horizontalen Unterlage steht.

Für optische Messungen, bei denen ein Lichtstrahl durch die beiden Seitenflächen und die im Innenraum angeordnete Flüssigkeit hindurchtritt, ist es von Vorteil, wenn der Weg des Lichtstrahls durch die Küvette hindurch kurz ist. Der Abstand zwischen der Außenseite der ersten Seitenwand und der Außenseite der zweiten Seitenwand ist vorzugsweise kleiner als 8 mm, weiter vorzugsweise kleiner als 6 mm, weiter vorzugsweise kleiner als 5 mm. Die Wandstärke der Küvette im Bereich der Seitenwand ist vorzugsweise kleiner als 1,5 mm, weiter vorzugsweise kleiner als 1,2 mm, weiter vorzugsweise kleiner als 0,9 mm. Parallel zur Seitenfläche kann der Innenraum der Küvette sich beispielsweise über 8 mm bis 15 mm erstrecken. Der Innenraum der Küvette kann eine Höhe zwischen 20 mm und 30 mm haben.

Damit das Licht beim Hindurchtreten durch die Seitenwand möglichst wenig beeinflusst wird, kann die Seitenwand einen ebenen Abschnitt aufweisen. Der ebene Abschnitt erstreckt sich vorzugsweise über mindestens 50 %, weiter vorzugsweise über mindestens 60 %, weiter vorzugsweise über mindestens 80 % der Gesamtfläche, die die Küvette in der Ebene der Seitenwände aufspannt.

Um optische Messungen zu ermöglichen, besteht die Küvette vorzugsweise aus einem durchsichtigen Material, vorzugsweise einem durchsichtigen Kunststoff. Beispielsweise kann die Küvette aus Polystyrol bestehen. Die Küvette kann als einstückiges Spritzgussteil hergestellt sein.

Die Erfindung betrifft außerdem eine Anordnung aus einer Mehrzahl solcher Küvetten. Die Küvetten können in der von den Seitenwänden aufgespannten Längsrichtung hintereinander angeordnet sein. Die Küvettenanordnung kann mit einer in Längsrichtung ausgerichteten Führungseinrichtung versehen sein, sodass die Anordnung entlang der Führungseinrichtung so positioniert werden kann, dass ein Lichtstrahl in fester Position durch eine bestimmte Küvette hindurchtreten kann. Die Führungseinrichtung kann eine Rippe, vorzugsweise zwei Rippen umfassen, die sich in Längsrichtung der Küvettenanordnung erstrecken. Die Rippen können am Boden der Küvettenanordnung ausgebildet sein. Die Anordnung von Küvetten kann als einstückiges Spritzgussteil hergestellt sein.

Die Erfindung betrifft außerdem ein System aus einer Küvette und einer Kugel. Die Kugel steht vorzugsweise aus einem magnetischen Material, sodass sie durch einen außerhalb der Küvette angeordneten Magneten entlang der Laufschiene bewegt werden kann, um die Flüssigkeit zu Durchmischen. Beispielsweise kann die Kugel aus VA-Stahl bestehen.

Der Durchmesser der Kugel ist vorzugsweise so bemessen, dass der Abstand zwischen der Kugel und dem Boden der Küvette sich von dem Abstand zwischen der Kugel und der Seitenwand der Küvette um nicht mehr als 50 %, vorzugsweise um nicht mehr als 20 %, weiter vorzugsweise um nicht mehr als 10 % unterscheidet, wenn die Kugel in der Mitte der Küvette auf der Laufschiene aufliegt. In einer bevorzugten Ausführungsform sind die beiden Abstände gleich. Die Laufschiene der Küvette kann so gestaltet sein, dass die betreffenden Abstände konstant bleiben, wenn sich die Kugel entlang der Laufschiene bewegt. Der Abstand zwischen der Kugel und dem Boden der Küvette ist vorzugsweise kleiner als 50 %, weiter vorzugsweise kleiner als 20 %, weiter vorzugsweise kleiner als 10 % als der Durchmesser der Kugel. Bei einer Anordnung aus einer Mehrzahl von Küvetten ist bevorzugt für jede der Küvetten eine solche Kugel vorgesehen.

Die Erfindung betrifft ferner ein System aus einer Messvorrichtung und einer solchen Küvette. Ein Befüllelement der Messvorrichtung ist dazu ausgelegt, eine Flüssigkeitsmenge in die Küvette einzubringen. Die Messvorrichtung umfasst außerdem einen Antrieb für die Kugel, wobei der Antrieb dazu ausgelegt ist, die Kugel in einen definierten Zustand zu bringen, wenn die Küvette befüllt wird.

Ein definierter Zustand der Kugel beim Befüllen der Küvette ist deswegen wünschenswert, weil die Gefahr von Luftblasen besteht, wenn die Flüssigkeit beim Eintritt in die Küvette zunächst auf die Kugel trifft. Um dies zu vermeiden, kann die Kugel in eine Position gebracht werden, sodass die aus dem Befüllelement austretende Flüssigkeit nicht auf die Kugel, sondern direkt auf den Boden der Küvette trifft. Der Antrieb kann so ausgelegt sein, dass er die Kugel in eine solche Position bringt und dort hält. Vorzugsweise handelt es sich um eine außermittige Position in der Küvette, weiter vorzugsweise um eine Position an einem Ende der Laufschiene.

Alternativ ist es möglich, dass der Antrieb die Kugel in Bewegung hält, während die Flüssigkeit in die Küvette eingefüllt wird. Entstehende Luftblasen werden dann durch die Bewegung der Kugel unmittelbar wieder zerstört. Die Formulierung, dass die Kugel durch den Antrieb in einen definierten Zustand gebracht wird, umfasst sowohl die erste Variante, bei der die Kugel in eine feste Position gebracht wird, der sie nicht von der Flüssigkeit getroffen wird, als auch die zweite Variante, bei der die Kugel beim Befüllen in Bewegung gehalten wird.

Ein solches System hat eigenständigen erfinderischen Gehalt, auch ohne dass die Küvette mit einer Laufschiene ausgestattet ist.

In einer bevorzugten Ausführungsform umfasst der Antrieb einen Magneten, der auf einer Kreisbahn bewegt wird. Die Kugel kann der Bewegung des Magneten nur in der durch die Laufschiene vorgegebenen Richtung folgen. Die Kreisbewegung des Magneten wird also umgesetzt in eine Linearbewegung der Kugel. Es wird dadurch möglich, die Kugel beispielsweise mit einem einfachen Schrittmotor anzutreiben. Eine Messvorrichtung mit einem solchen Antrieb hat eigenständigen erfinderischen Gehalt, auch ohne dass die Kugel beim Befüllen in einen definierten Zustand gebracht wird und ohne dass die Laufschiene durch Vorsprünge in der Seitenwand der Küvette gebildet wird. Der Erfindungsgedanke kann mit jeder Art von linearer Laufschiene verwirklicht werden.

Die Kreisbahn, auf der sich der Magnet bewegt, kann in der horizontalen Ebene angeordnet sein. Der Durchmesser der Kreisbahn entspricht vorzugsweise im Wesentlichen dem Durchmesser der Küvette in Längsrichtung. Vorzugsweise erstreckt sich die Bewegung der Kugel über mindestens 50 %, vorzugsweise mindestens 60 %, weiter vorzugsweise mindestens 80 % der Länge der Laufschiene. Die Drehzahl kann zwischen 0,1 Umdrehungen/Sekunde und 17 Umdrehungen/Sekunde, bevorzugt zwischen 0,2 Umdrehungen/Sekunde und 10 Umdrehungen/Sekunde liegen. Vorzugsweise ist der Antrieb dazu ausgelegt, den Magneten zum Mischen der Flüssigkeit auf einer entsprechenden Kreisbahn zu bewegen. Der Antrieb kann außerdem zum Rühren der Flüssigkeit verwendet werden.

Die Messvorrichtung kann einen Messkanal umfassen, der sich von einer Lichtquelle durch die in passender Position angeordnete Küvette hindurch bis zu einem Lichtsensor erstreckt.

Die Messvorrichtung kann außerdem eine Steuerung umfassen, die dazu ausgelegt ist, die Funktionen der Messvorrichtung in geeigneter Weise anzusteuern. Insbesondere kann die Steuerung dazu ausgelegt sein, das Zusammenspiel zwischen dem Befüllelement und dem Antrieb zu steuern. Es kann dazu zunächst der Antrieb so angesteuert werden, dass er die Kugel in den definierten Zustand bringt. Anschließend kann das Befüllelement angesteuert werden, sodass es eine bestimmte Menge Flüssigkeit in die Küvette einfällt. Nach dem Befüllen ist die Kugel vorzugsweise vollständig in die Flüssigkeit eingetaucht, weiter vorzugsweise ist der Füllstand in der Küvette wenigstens doppelt so hoch wie der Durchmesser der Kugel. Dadurch wird es möglich, den Messkanal, der sich durch die Flüssigkeit hindurch erstreckt, oberhalb der Kugel anzuordnen, sodass der Messvorgang unbeeinflusst von einer Bewegung der Kugel bleibt. Die Füllmenge der Flüssigkeit kann beispielsweise zwischen 100 µl und 200 µl, vorzugsweise bei weniger als 180 µl liegen.

Die Messvorrichtung kann ferner eine Zuführeinheit umfassen, die dazu ausgelegt ist, eine Kugel zu der Küvette zuzuführen. Es kann ein Sensor vorgesehen sein, der das Vorhandensein der Kugel prüft. Die Messvorrichtung kann eine Heizung umfassen, um die Flüssigkeit in der Küvette zu erwärmen. Insbesondere kann die Heizung dazu ausgelegt sein, die Flüssigkeit auf eine Temperatur zwischen 35 °C und 40 °C zu erwärmen. Die Heizung kann so ausgelegt sein, dass sich das Erwärmen der Flüssigkeitsmenge über einen Zeitraum zwischen 1 Minute und 3 Minuten erstreckt.

Die Messvorrichtung kann eine Mehrzahl von Messstationen umfassen, sodass mehrere Proben gleichzeitig untersucht werden können. Vorzugsweise umfasst jede Messstation einen Antrieb und einen Messkanal. Es kann eine Küvettenanordnung verwendet werden, in der mehrere Küvetten so miteinander verbunden sind, dass jeder Messkanal sich durch eine Küvette hindurch erstreckt. Wenn der Abstand zwischen den Messkanälen doppelt so groß ist wie der Abstand zwischen zwei benachbarten Küvetten der Küvettenanordnung, können mit der Küvettenanordnung zwei Messläufe durchgeführt werden. Im ersten Messlauf wird jede zweite Küvette untersucht. Anschließend wird die Küvettenanordnung um die Länge einer Küvette verschoben, sodass sich die Messkanäle durch die benachbarte Kanüle erstrecken, die im ersten Messlauf unbenutzt war.

Die Messvorrichtung kann insbesondere mit dem Ziel verwendet werden, die Thrombozyten-Reaktion in Vollblut oder in Blutplasma zu untersuchen. Der Ablauf ist wie folgt. Zunächst wird eine Küvette in die Messvorrichtung eingesetzt und eine Kugel in die Küvette eingebracht. Die Küvette wird so positioniert, dass der Antrieb auf die Kugel wirken kann. Die Kugel wird in einen definierten Zustand gebracht. Das Vollblut oder das Blutplasma wird in die Küvette eingebracht. Das Blut bzw. Blutplasma wird erwärmt. Beim Erwärmen steht die Kugel vorzugsweise still. Ein Reagenz wird hinzugefügt. Die Kugel wird mit dem Antrieb in Bewegung versetzt, um die Flüssigkeit in der Küvette zu durchmischen. Das durch die Flüssigkeit hindurchtretende Licht wird gemessen.

Bei der Messung ergibt sich üblicherweise, dass infolge einer Agglomeration der Thrombozyten die Trübung während der Messdauer abnimmt, also zunehmend mehr Licht durch die Flüssigkeit hindurchtreten kann. Der Verlauf der Lichtstärke über der Zeit kann aufgezeichnet werden. Eine Messung kann sich beispielsweise über einen Zeitraum zwischen 10 Minuten und 15 Minuten erstrecken. Aus der Aufzeichnung lassen sich Rückschlüsse über den Zustand des Bluts bzw. Blutplasmas ziehen. Vorzugsweise umfasst die Messvorrichtung fünf Messstationen, sodass das Blut eines Patienten mit fünf verschiedenen Reagenzien gleichzeitig untersucht werden kann. Wird eine Anordnung aus zehn Küvetten verwendet, so kann die betreffende Untersuchung mit derselben Küvettenanordnung nacheinander für zwei verschiedene Patienten durchgeführt werden.

Die Thrombozyten in dem Blut bzw. Blutplasma reagieren empfindlich auf Abweichungen im Messablauf. Die Messvorrichtung ist deswegen vorzugsweise so eingerichtet, dass der gesamte Messablauf vollautomatisch erfolgt, um Abweichungen aufgrund menschlicher Eingriffe zu vermeiden. Die Messvorrichtung kann dazu eine Aufnahme aufweisen, in die die Probe eingesetzt wird. Vorzugsweise wird die Probe für eine bestimmte Zeit nicht bewegt, sodass das Blut bzw. Blutplasma zur Ruhe kommen kann. Anschließend kann die Aufnahme für eine leichte Durchmischung des Vollbluts einmal oder mehrfach langsam geschwenkt werden. Bei Blutplasma entfällt das Schwenken vorzugsweise. Mit einer Nadel kann in die Probe eingestochen werden, um eine bestimmte Menge der Flüssigkeit zu entnehmen. Die Flüssigkeit kann in die Küvette eingebracht werden, um dort gemäß dem beschriebenen Ablauf untersucht zu werden. Hier ist es für die Reproduzierbarkeit von besonderer Bedeutung, dass die Kugel sich auf der Laufschiene bewegt, weil der Bereich, in dem durch die Kugel lokal ein großer Druck ausgeübt wird, dadurch klein gehalten wird. Außerdem ist der gleichmäßige Abstand zwischen der Kugel und den Seitenflächen sowie Bodenflächen der Küvette von Bedeutung, durch den erreicht wird, dass die beim Mischen auftretende Scherung in der Flüssigkeit möglichst gleichmäßig ist. Ferner ergibt sich aus der erfindungsgemäßen Form der Küvette, dass die Messung mit der sehr geringen Flüssigkeitsmenge von etwa 150 µl möglich ist. Im Unterschied dazu benötigt man bei konventionellen Messungen etwa 300 µl bis 400 µl der Flüssigkeit. Gerade bei Proben, die von Kindern stammen, steht häufig nicht so viel Flüssigkeit zur Verfügung.

Die Erfindung betrifft außerdem ein Verfahren zum Untersuchen einer Flüssigkeit, insbesondere von Blut bzw. Blutplasma, bei dem Licht durch eine in einer Küvette angeordnete Menge der Flüssigkeit hindurch geleitet wird und dass durch die Flüssigkeit hindurchgetretene Licht aufgezeichnet wird. Das Verfahren kann unter Verwendung einer erfindungsgemäßen Küvette und/oder einer erfindungsgemäßen Messvorrichtung durchgeführt werden. Das Verfahren kann mit Merkmalen weitergebildet werden, die im Zusammenhang der erfindungsgemäßen Küvette und/oder im Zusammenhang der erfindungsgemäßen Messvorrichtung beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: diverse Ansichten einer erfindungsgemäßen Küvettenanordnung;
- Fig. 2a:: eine vergrößerte Schnittdarstellung von Teilen einer Küvette aus Fig. 1;
- Fig. 2b:: eine vergrößerte Schnittdarstellung der Küvette aus Fig. 2a mit einer Kugel;
- Fig. 3:: eine schematische Darstellung einer erfindungsgemäßen Messvorrichtung in einer Ansicht von der Seite und einer Ansicht von oben;
- Fig. 4:: eine schematische Darstellung einer erfindungsgemäßen Messvorrichtung zum Durchführen einer optischen Messung an der Flüssigkeit; und
- Fig. 5:: eine seitliche Ansicht der Messvorrichtung aus Fig. 4.

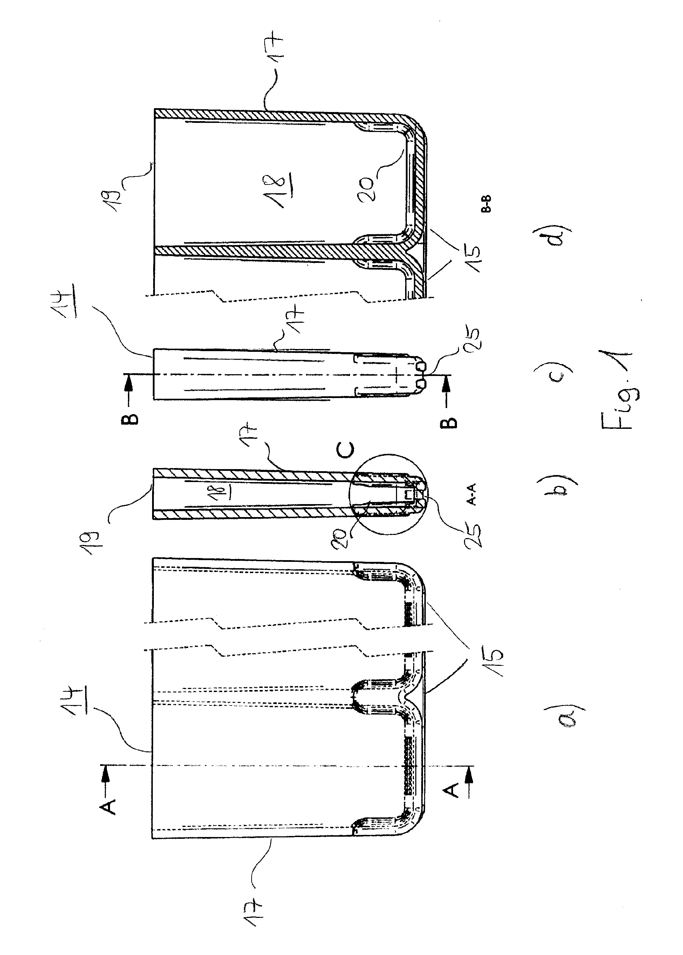

Eine erfindungsgemäße Küvette 14 ist als Teil einer erfindungsgemäßen Küvettenanordnung 15 in den Figuren 1a und 1c in Draufsicht und in den Figuren 1b und 1d als Schnittdarstellung gezeigt. Eine Küvette 14 umfasst einen von einem Küvettengehäuse 17 umschlossenen Innenraum 18, der über einen Einlass 19 in Kontakt mit der Umgebung ist. In einem unteren Bereich des Innenraums 18 erstreckt sich eine Laufschiene 20, die mit dem Küvettengehäuse 17 verbunden ist.

In dieser Ausführungsform ist die Laufschiene 20 an beiden Enden nach oben gekrümmt und folgt dem Verlauf des Küvettengehäuses 17. Außerdem ist der Innenraum 18 von unten nach oben leicht aufgeweitet. Ferner weist das Küvettengehäuse 17 an einer unteren Außenseite eine Führungseinrichtung 25 auf, welche sich über alle Küvetten 14 eines Küvettenanordnungs 15 erstreckt.

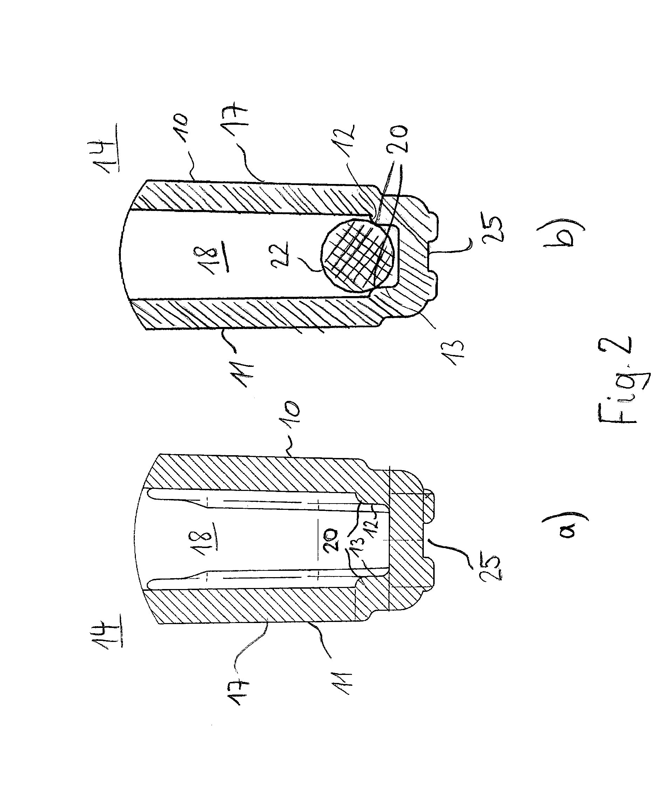

In einer vergrößerten Darstellung in Fig. 2 ist der untere Teil einer erfindungsgemäßen Küvette 14 gezeigt. Wie in Fig. 2a gezeigt, erstreckt sich die Laufschiene 20 innerhalb des Innenraums 18 entlang einer unteren Seite des Küvettengehäuses 17 und verläuft weiter entlang einer abgerundeten Kante und einer weiteren Seite des Küvettengehäuses 17. In Fig. 2b ist eine Kugel 22 mit definiertem Durchmesser in den Innenraum 18 so angeordnet, dass die Kugel 22 auf der Laufschiene 20 aufliegt. Die Kugel 22 ist dabei, abgesehen von den dargestellten Auflagepunkten (Kontaktbereich) auf der Laufschiene 20, nicht in Kontakt mit dem Küvettengehäuse 17. Der Abstand zwischen dem untersten Punkt der Kugel 22 und dem Küvettengehäuse 17 ist identisch zu dem Abstand zwischen den seitlichsten Punkten der Kugel 22 und der Seitenwand des Küvettengehäuses 17.

Durch die Krümmung an den Enden der Laufschiene 20 ist sichergestellt, dass die Kugel 22 nicht gegen die Stirnwand der Küvette laufen kann. Wenn sich die Kugel 22 mit hoher Geschwindigkeit entlang der Laufschiene 20 bewegt, kann sie an den Enden hochlaufen und wird so abgebremst, bevor sie in Kontakt mit dem Küvettengehäuse 17 kommt.

In Fig. 2b ist ersichtlich, dass ein imaginärer erster Kreis, dessen Durchmesser etwas größer ist als der Durchmesser der Kugel 22 die beiden Vorsprünge 12, 13 der Laufschiene 20 schneidet. Hingegen schneidet ein imaginärer zweiter Kreis, der etwas kleiner ist als die Kugel 22, die beiden Vorsprünge nicht.

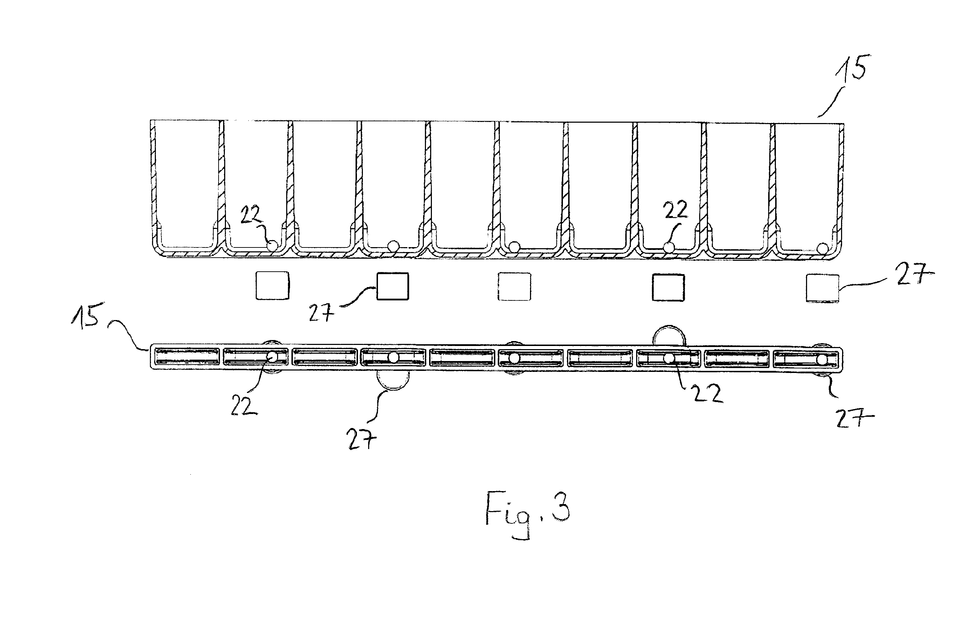

Fig. 3 zeigt einen Küvettenanordnung 15, bestehend aus zehn Küvetten 14, wobei die Laufschienen 20 der einzelnen Küvetten 14 in einer Reihe angeordnet sind. In jeder zweiten Küvette 14 ist eine Kugel 22 angeordnet. Die Position der Kugeln 22 ergibt sich aus den jeweils außerhalb der Küvettengehäuse 17 angeordneten Magneten 27, welche mit den ferromagnetischen Kugeln 22 wechselwirken.

Durch die magnetische Wechselwirkung ist es möglich, die Kugel 22 in einem definierten Zustand, zum Beispiel an einem Ende der Laufschiene 20, zu halten. Ein solcher definierter Zustand der Kugel 22 ist besonders beim Einbringen einer Flüssigkeit in den Innenraum 18 (nicht gezeigt) mit einem nicht gezeigten Befüllelement vorteilhaft. So kann vermieden werden, dass ein Pipettierstrahl direkt auf die Kugel 22 auftrifft und dadurch unnötige Turbulenzen in der Flüssigkeit entstehen. Darüber hinaus ist es möglich, dass sich die Kugel 22 in einem definierten Zustand befindet, indem sie sich langsam oder mit konstanter Geschwindigkeit entlang der Laufschiene 20 bewegt, wie es im Zusammenhang mit dem erfindungsgemäßen Verfahren erklärt ist.

Das erfindungsgemäße Verfahren zum Rühren einer Flüssigkeit in einer Küvette kann anhand der abgebildeten Zustände der Kugeln 22 und der Magnete 27 nachvollzogen werden. Im oberen Teil der Fig. 3 ist die Küvettenanordnung 15, mit Kugeln 22 und Magneten 27 von der Seite gezeigt, während der gleiche Zustand im unteren Teil der Fig. 3 aus der Vogelperspektive zu sehen ist. Der Magnet 27 ist in dieser Ausführungsform in Form eines Zylinders dargestellt, der von der Seite betrachtet einen rechteckigen und von oben betrachtet einen kreisförmigen Querschnitt aufweist.

Bei einer vollen Umdrehung gemäß des erfindungsgemäßen Verfahrens bewegt sich der Magnet 27 einmal entlang einer Kreisbahn, während sich die Kugel 22 entlang der Laufschiene 20 einmal hin und zurück bewegt. Betrachtet man die Zustände in dieser beispielhaften Darstellung von links nach rechts, so ist die Kugel 22 zunächst auf der rechten Seite der Laufschiene 20 angeordnet, während der Magnet 27 direkt unterhalb der Kugel 22 unter der rechten Seite der Laufschiene 20 platziert ist. Bewegt sich der Magnet 27 entlang eines Viertels einer Kreisbahn im Uhrzeigersinn, rollt die ferromagnetische Kugel 22 bis zur Mitte der Laufschiene 20 und weiter bis zum linken Rand der Laufschiene 20, wenn der Magnet 27 das zweite Viertel auf der Kreisbahn zurückgelegt hat. Der Rückweg erfolgt analog, bis sowohl der Magnet 27 als auch die ferromagnetische Kugel 22 ihre Ausgangspositionen wieder erreicht haben.

Der Magnet 27 kann von einem nicht gezeigten Schrittmotor schrittweise angetrieben werden und die Kugel 22 kann teilweise oder vollständig in eine nicht gezeigte Probe getaucht sein. Die wiederholte Bewegung der ferromagnetischen Kugel 22 erzeugt dann eine Pumpwirkung in der Probe, die dafür sorgt, dass die Probe effizient durchmischt wird.

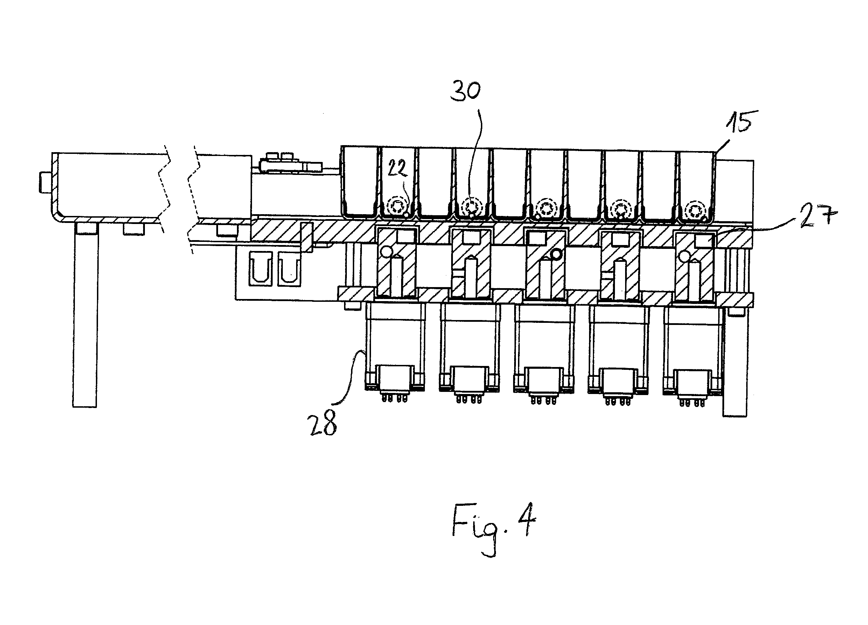

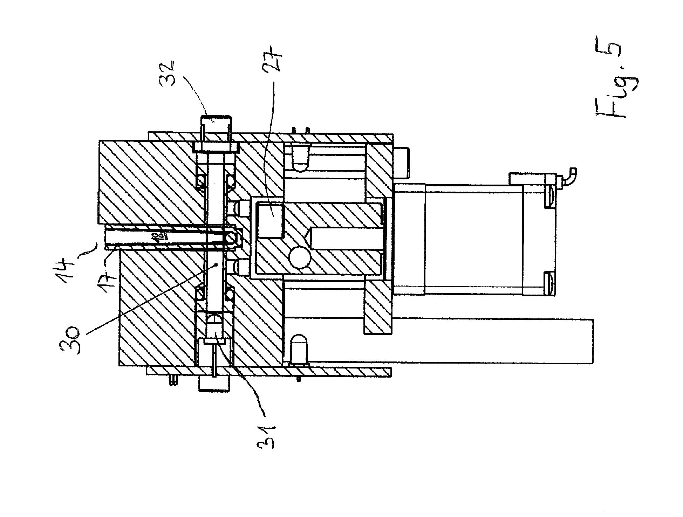

In Fig. 4 und 5 ist eine erfindungsgemäße Maschine zur Bestimmung der Trübung einer Flüssigkeit gezeigt, die dazu ausgelegt ist, das erfindungsgemäße Verfahren durchzuführen und mit einer erfindungsgemäßen Küvettenanordnung 15 befüllt zu werden. Die Küvettenanordnung 15 weist, wie in Fig. 3 gezeigt, in jeder zweiten Küvette eine Kugel 22 auf. Die Anordnung der Kugeln 22 und die Ausrichtung der darunter befindlichen Magnete 27 ist insgesamt identisch zu dem Beispiel aus Fig. 3. Die Führungseinrichtung 25 der Küvettenanordnung 15 wirkt mit der Maschine so zusammen, dass die Küvettenanordnung horizontal verschoben werden kann und entsprechend den Magneten 27 ausgerichtet werden kann. Zusätzlich bilden in dieser Ausführungsform fünf in einer Reihe angeordnete Schrittmotoren 28, die separat angesteuert werden können, einen Teil der erfindungsgemäßen Maschine. Die Schrittmotoren 28 sind dazu ausgelegt, jeweils einen Magneten 27 entlang einer Kreisbahn zu bewegen. Dadurch ist ein simultanes Rühren in fünf Küvetten gleichzeitig möglich.

Die erfindungsgemäße Maschine ermöglicht es, die Trübung einer Probe während des Rührens oder nachdem das Rühren abgeschlossen ist, vollautomatisch zu bestimmen. Dazu ist ein Messkanal 30 vorgesehen, der sich durch die Küvette 14 erstreckt. Der Messkanal 30 ist bevorzugt so angeordnet, dass er sich mittig und in einer Höhe, in der der Innenraum 18 vollständig mit einer Probe gefüllt ist, erstreckt. Während der Messung sollte die Kugel 22 so angeordnet sein oder in einem Bereich bewegt werden, der den Messkanal 30 nicht verdeckt.

In Fig. 5 ist eine seitliche Schnittdarstellung der erfindungsgemäßen Maschine zur Bestimmung der Trübung einer Flüssigkeit gezeigt. Der Messkanal 30 erstreckt sich zwischen einer Lichtquelle 31 und einem Photodetektor 32 in der Form, dass das Licht die Küvette 14 in einem Bereich oberhalb der Kugel 22 passiert. Von der Lichtquelle 31 ausgesandtes Licht tritt durch das Küvettengehäuse 17 in den Innenraum 18 der Küvette 14, transmittiert durch die Probe und tritt erneut durch das Küvettengehäuse 17 aus der Küvette 14 aus, um schließlich von einem Photodetektor 32 aufgezeichnet zu werden.

Der Innenraum 18 sollte im Bereich des Messkanals 30 eine geringe Ausdehnung aufweisen, damit das Licht von der Probe nicht vollständig absorbiert wird. Im Bereich des Messkanals 30 sollte das Küvettengehäuse 17 eine Wandstärke von 2 mm nicht übertreffen, um die Messergebnisse nicht zu verfälschen. Der Messkanal 30 sollte ferner hinreichend groß sein, um einen repräsentativen Bereich der Probe abzudecken.

Die erfindungsgemäße Maschine ermöglicht eine Messung der Trübung einer Probe oder verschiedener Proben in fünf Messkanälen 30 gleichzeitig. Im Anschluss an die Messung, kann die Küvettenanordnung 15, beispielsweise um eine Küvettenposition, horizontal verschoben werden, um die fünf bisher unbenutzten Küvetten 14 entsprechend der Schrittmotoren 28 und Messkanäle 30 auszurichten und das Rühren gemäß des erfindungsgemäßen Verfahrens und die Messung der Trübung zu wiederholen.

## Patentansprüche

1. Küvette zum Rühren einer Flüssigkeit, umfassend einen Innenraum (18) zur Aufnahme der Flüssigkeit und eine in dem Innenraum (18) ausgebildete Laufschiene (20) für eine Kugel (22), **dadurch gekennzeichnet, dass** die Laufschiene (20) von zwei Vorsprüngen (12, 13) gebildet wird, die von gegenüberliegenden Seitenwänden (10, 11) der Küvette (14) in den Innenraum (18) vorspringen.

2. Küvette nach Anspruch 1, **dadurch gekennzeichnet, dass** ein imaginärer erster Kreis, der senkrecht zur Laufschiene (20) ausgerichtet ist und der am Boden des Innenraums (18) sowie an den Seitenwänden (10, 11) des Innenraums (18) anliegt die beiden Vorsprünge (12, 13) schneidet, während ein kleinerer, konzentrischer imaginärer zweiter Kreis die Vorsprünge (12, 13) nicht schneidet, wobei der Durchmesser des zweiten Kreises mindestens 50 %, vorzugsweise mindestens 60 %, weiter vorzugsweise mindestens 80 % des Durchmessers des ersten Kreises ist.

3. Küvette nach Anspruch 1 oder 2, **dadurch gekennzeichnet, dass** der Kontaktbereich des Vorsprungs (12, 13) im Querschnitt betrachtet eine konvexe Form hat.

4. Küvette nach einem der Ansprüche 1 bis 3, **dadurch gekennzeichnet, dass** die Laufschiene (20) an einem Ende oder an beiden Enden nach oben umgelenkt ist.

5. Küvette nach einem der Ansprüche 1 bis 4, **dadurch gekennzeichnet, dass** die Seitenwände (10, 11) der Küvette (14) im Wesentlichen parallel zueinander sind.

6. Küvette nach einem der Ansprüche 1 bis 5, **dadurch gekennzeichnet, dass** die Seitenwände (10, 11) der Küvette (14) in der vertikalen Dimension einen Winkel zwischen 0,2° und 5°, vorzugsweise zwischen 0,5° und 2° einschließen.

7. Küvette nach einem der Ansprüche 1 bis 6, **dadurch gekennzeichnet, dass** der Abstand zwischen der Außenseite der ersten Seitenwand (10) und der Außenseite der zweiten Seitenwand (11) kleiner als 8 mm, vorzugsweise kleiner als 6 mm, weiter vorzugsweise kleiner als 5 mm ist.

8. System aus einer Küvette (14) nach einem der Ansprüche 1 bis 7 und einer Kugel (22), **dadurch gekennzeichnet, dass** die auf der Laufschiene (20) aufliegende Kugel (22) einen Abstand zur Bodenfläche der Küvette (14) hat, der sich von dem Abstand zur Seitenwand (10, 11) um nicht mehr als 50 %, vorzugsweise um nicht mehr als 20 %, weiter vorzugsweise um nicht mehr als 10 % unterscheidet.

9. System aus einer Messvorrichtung und einer Küvette (14) nach einem der Ansprüche 1 bis 7, **dadurch gekennzeichnet, dass** die Messvorrichtung folgende Elemente umfasst:
a. ein Befüllelement, das dazu ausgelegt ist, eine Flüssigkeitsmenge in die Küvette (14) einzubringen;
b. einen Antrieb (28) für eine in der Küvette (14) angeordnete Kugel (22), wobei der Antrieb (28) dazu ausgelegt ist, die Kugel (22) in einen definierten Zustand zu bringen, wenn das Befüllelement die Küvette (14) befüllt.

10. System nach Anspruch 9, **dadurch gekennzeichnet, dass** in dem definierten Zustand die Kugel (22) in einer festen Position angeordnet ist oder die Kugel (22) in Bewegung gehalten wird.

11. System nach Anspruch 9 oder 10, **dadurch gekennzeichnet, dass** der Antrieb (28) einen auf einer Kreisbahn bewegten Magneten umfasst.

12. System nach einem der Ansprüche 9 bis 11, **dadurch gekennzeichnet, dass** die Messvorrichtung einen sich von einer Lichtquelle (31) bis zu einem Lichtsensor (32) erstreckenden Messkanal umfasst, der sich oberhalb der auf der Laufschiene (20) aufliegenden Kugel (22) durch die Küvette (14) hindurch erstreckt.

13. System nach einem der Ansprüche 9 bis 12, **dadurch gekennzeichnet, dass** die Messvorrichtung eine Mehrzahl von Messstationen umfasst, wobei jede Messstation einen Messkanal (31, 32) und einen Antrieb (28) aufweist.

14. Verfahren zum Untersuchen einer Flüssigkeit, bei dem ein Lichtstrahl durch die in einer Küvette angeordnete Flüssigkeit geleitet wird und der transmittierte Teil des Lichts gemessen wird, **dadurch gekennzeichnet, dass** die Küvette (14) nach einem der Ansprüche 1 bis 7 ausgebildet ist.
